Europäisches Patentamt

European Patent Office

Office européen des brevets

(11) Publication number: **0 410 921 A2**

# EUROPEAN PATENT APPLICATION

(21) Application number: 90500072.5

(22) Date of filing: 24.07.90

(51) Int. Cl.5: **A61M 37/00**

(30) Priority: 28.07.89 ES 8903287

(43) Date of publication of application:
**30.01.91 Bulletin 91/05**

(84) Designated Contracting States:
**CH DE FR GB IT LI**

(71) Applicant: **Aguilera Franco, Maria**
**C. Muntaner, 280**
**E-08021 Barcelona(ES)**

(72) Inventor: **Aguilera Franco, Maria**
**C. Muntaner, 280**
**E-08021 Barcelona(ES)**

(74) Representative: **Ponti Sales, Adelaida**
**Paseo de Gracia, 33**
**E-08007 Barcelona(ES)**

(54) **Dressing for transcutaneous application of active substances for therapeutic or cosmetic purposes.**

(57) It comprises a permeable or semi-permeable membrane (1) in contact with the skin, a receptacle (2) for active substances in contact with that membrane, an impermeable layer (3) which surrounds the active substances receptacle (2) to the exterior, and an adhesive layer (4) for fixing the dressing to the skin, characterized in that the active substances receptacle (2) is formed by a fibrous network (6, 7) which, when impregnated with the active substances, retains them in a homogeneous way in its mesh.

Liberation of the substances in contact with the skin is achieved in a uniform way, thus facilitating stable and controlled absorption.

This is of application in cosmetics and medicine.

FIG. 2

# DRESSING FOR TRANSCUTANEOUS APPLICATION OF ACTIVE SUBSTANCES FOR THERAPEUTIC OR COSMETIC PURPOSES

The present invention refers to a dressing for transcutaneous application of active substances for therapeutic or cosmetic purposes. When applied locally to the zone to be treated, it permits the passage of active therapeutic or cosmetic substances through the skin.

## BACKGROUND OF THE INVENTION

There are known dressings which comprise a permeable or semi-permeable membrane in contact with the skin, an active substances receptacle in contact with that membrane, an impermeable layer which surrounds the active substances receptacle to the exterior and with an adhesive layer to fix the dressing to the skin.

Once the dressing has been attached to the skin by means of the adhesive layer, the active substances penetrate into the skin from their receptacle and through the permeable or semi-permeable membrane.

European patent 0 298 297 describes a dressing of this type which is characterized by the active substance reserve consisting in a system of several chambers - that is, a multi-chamber system - in which the chambers are interconnected.

When one chamber runs out of the active substances it holds, it receives the substance from another interconnected chamber. The active substance penetrates the skin by the force of gravity.

This characteristic presents limitations with respect to the distribution of the active substance in the receptacle and in uniform application to the skin.

## DESCRIPTION OF THE INVENTION

The dressing of the invention manages to resolve these disadvantages.

The dressing object of the invention is characterized by the fact that the active substances receptacle is formed by a fibrous network which, when impregnated with the active substances, retains them in its mesh in a homogeneous way, facilitating stable and controlled absorption.

Advantageously, the edges of the fibrous mesh are sealed in order to prevent escape of the substances contained and protected with an adhesive layer to the edges of which is applied the adhesive for attachment thereof.

Optionally, the dressing also includes a protective layer of anti-adherent material arranged beneath the adhesive layer and the permeable or semi-permeable membrane, in order to avoid dispersion and escape of the active substances. The protective layer may be of siliconed or waxed paper or of any other type of anti-adherent material.

That protective layer avoids any possible overflowing due to pressure, the substances being retained sheltered from oxidation and dehydration. This protective layer, anti-adherent to the skin, seals the dressing by adhering to it, but is easily detachable at the time of use.

The dressing can be presented under a great variety of shapes, such as rectangular or rounded, or can be adapted to the anatomy of the zones to be treated.

The active substances may be presented in the form of gels, ointments, creams and hydroalcoholic or oil-based solutions.

The substances to be applied for the cosmetic or medicinal treatments of transdermal application may be of vegetable, chemical, pharmacological or biological origin. For example, and without exclusion of others, they may be biological products or derivatives of same, or chemical products of medical or cosmetic efficacy; they may also be vegetable tinctures or extracts, or chemical derivatives of the same vegetable substances. Use shall be made of aqueous, hydroalcoholic or liposophyllic excipients suitable to the substance to be employed and to the cosmetic or medical action which it is wished to obtain.

The fibrous network may similarly be of various materials, such as polyester, cellulose, polyamide or polyamidic derivative.

An important quality of this fibre consists in it not containing synthetic resins or any type of chemical bonding agents which could interfere with the chemical and biological action of the product.

The dressing of the invention permits absorption of the active substances in an occlusive manner, thus avoiding evaporation, dessication and oxidation of the active substances, as occurs with the known applications.

As this is a prolonged local application, higher transcutaneous absorption is achieved over larger surface areas, so that skin or subcutaneous tissue nutrient, stimulant or relaxing products to be used are of basically surface and local effect. Great local concentration is thus achieved, with negligible minimum general action.

The impermeable layer avoids occurrence of evaporation and oxidation of the water-soluble, hydroalcoholic or liposoluble ingredients, thus maintaining stability of action.

Another advantage of the dressing of the invention in comparison with others of local action consists in that the fixation and form of product retention is uniformly spread thanks to the fibrous network, offering concentration stability and homogeneous product distribution, thus allowing stable and uniform absorption throughout the time of application, which may vary in accordance with the medicament and the therapeutic effect sought.

Another advantage is its adaptation to the anatomical shapes to be treated, thus achieving an intensely local therapeutic effect and minimum general action and avoiding the side-effects so frequent in treatments carried out via other channels.


BRIEF DESCRIPTION OF THE DRAWINGS


For a better understanding of all that has been outlined above, some drawings are attached which, schematically and solely by way of non-restrictive example, show a practical case of embodiment.

In those drawings, figure 1 is a perspective view of the dressing of the invention; figure 2 is a section view of dressing, showing an embodiment of the interior fibrous network; figure 3 is a section view of another embodiment of the dressing with another interior fibrous network; figures 4 to 7 show further embodiments of the interior fibrous network.


DESCRIPTION OF PREFERRED EMBODIMENTS


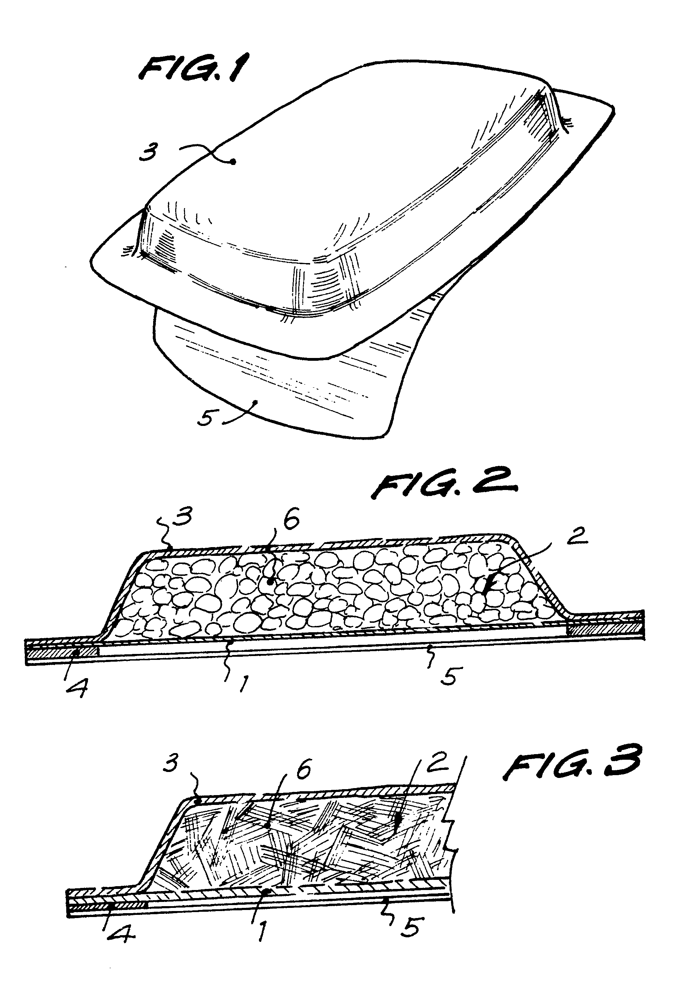

As the figures show, the dressing of the invention comprises a permeable or semi-permeable membrane 1 in contact with the skin, a receptacle 2 for active substances in contact with that membrane 1, an impermeable layer 3 which surrounds the active substances receptacle 2 to the exterior, and an adhesive layer 4 for fixing the dressing to the skin, and a protective layer 5 of anti-adherent material arranged below the adhesive layer and the permeable or semi-permeable membrane, to avoid dispersion and loss of the active substances. Figure 1 shows the protective layer 5 as the dressing is extracted to permit contact of the permeable layer 1 with the skin.

As shown in figures 2 and 3, the receptacle 2 is formed by aa fibrous network which, impregnated with the active substances, retains them in a homegeneous way in its meshes, achieving uniform release of the substances in contact with the skin, thus facilitating stable and controlled absorption.

Figure 2 shows a fibrous network 6 with structure of granular appearance, while figure 3 shows another variant of the fibrous structure 6 formed by fibres laid out in an irregular manner.

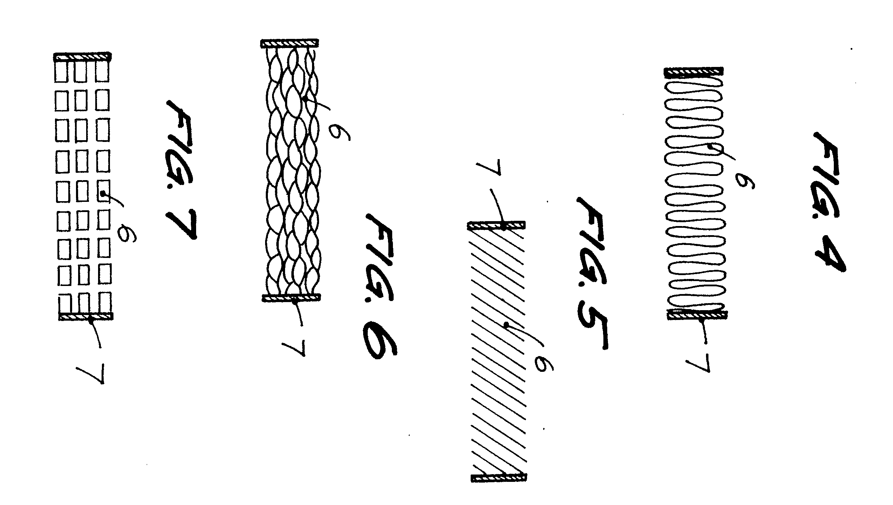

Figures 4 to 7 show other embodiments of the fibrous network 6 inside which are located the active substances. The figures also show the edges of sealing 7. The figures show representations of some of the possible embodiments, but it is understood that the fibrous network may be of any other type.


Claims


1. Dressing for transcutaneous application of active substances, of the type which comprises a permeable or semi-permeable membrane (1) in contact with the skin, a receptacle (2) for active substances in contact with that membrane, an impermeable layer (3) which surrounds the active substances receptacle (2) to the exterior, and an adhesive layer (4) for fixing the dressing to the skin, characterized in that the active services receptacle (2) is formed by a fibrous network (6) which, when impregnated with the active substances, retains them in its mesh in a uniform way, facilitating stable and controlled absorption.

2. Dressing according to claim 1, characterized in that the edges (7) of the fibrous network (6) are sealed in order to prevent escape of the substances it contains and protected with an impermeable layer to the edges of which is applied the adherent for the securing thereof.

3. Dressing according to claim 1, characterized in that it includes a protective layer (5) of anti-adherent material arranged below the adhesive layer (4) and the permeable or semi-permeable membrane (1), in order to avoid dispersion and loss of the active substances.

4. Dressing according to claim 1, characterized in that the active substances which impregnate the fibrous network (6) are tinctures and aqueous solutions of vegetable extracts or chemical derivatives of vegetable products.

5. Dressing according to claim 1, characterized in that the active substances which impregnate the fibrous layer (6) are biological products or derivatives of same.

6. Dressing according to claim 1, characterized in that the active substances which impregnate the fibrous layer (6) are chemical products of medical or cosmetic efficacy.

7. Dressing according to claim 1, characterized in that the fibrous network (6) is of polyester.

8. Dressing according to claim 1, characterized in that the fibrous network (6) is of polyamide.

9. Dressing according to claim 1, characterized in that the fibrous network (6) is of cellulose.

10. Dressing according to claim 1, characterized in that the fibrous network (6) is a polyamidic derivative.

FIG.1

FIG.2

FIG.3

*FIG. 4*

6

7

*FIG. 5*

7

6

*FIG. 6*

6

7

*FIG. 7*

6

7